# EUROPEAN PATENT SPECIFICATION

(11) **EP 2 403 601 B1**
(45) Date of publication and mention of the grant of the patent: **04.05.2016**
(21) Application number: 10714501.3
(22) Date of filing: 02.03.2010
(51) Int. Cl.: A61N 7/00, A61H 7/00, A61B 18/14

(54) **LOW- AND MID-FREQUENCY ULTRASOUND DEVICE WITH ENHANCED CAVITATION EFFECT IN COMBINATION WITH RADIAL IN-DEPTH SKIN THERAPY**
NIEDER-UND MITTELFREQUENTE ULTRASCHALLVORRICHTUNG MIT VERBESSERTER KAVITATIONSWIRKUNG IN KOMBINATION MIT RADIALER TIEFENTHERAPIE DER HAUT
DISPOSITIF À ULTRASON À FRÉQUENCES FAIBLE ET MOYENNE EN COMBINAISON AVEC UNE THÉRAPIE DE LA PEAU EN PROFONDEUR

(30) Priority: 03.03.2009 SI 200900060
(43) Date of publication of application: 11.01.2012
(73) Proprietor: Iskra Medical, D.O.O., 1000 Ljubljana (SI)
(72) Inventor: JELENC, Jure, SI-4244 Podnart (SI); JELENC, Joze, SI-4244 Podnart (SI)
(74) Representative: Zacco GmbH
(86) International application number: PCT/SI2010/000009
(87) International publication number: WO 2010/101532

(56) References cited:
- WO-A1-2007/093998
- WO-A2-2009/098606
- US-A1- 2006 241 485
- US-A1- 2008 195 000

## Description

### Field of the invention

The invention relates to a device for the treatment of subcutaneous fat tissue, specifically to a device that uses low- and mid-frequency ultrasound waves in combination with in-depth skin therapy.

### Technical problem

The technical problem solved by this device is the design and construction of a device that includes an applicator for destruction of subcutaneous fat tissue using low- and mid-frequency ultrasound in combination with in-depth vacuum therapy. Additionally the device described in the invention, must be able to filter any excess gel absorbed into the device's interior during therapy. Cavitation is the process by which subcutaneous fat cells are selectively and non-surgically removed by applying concentrated ultrasound energy to a certain tissue depth. The skin surface, veins, nerves and muscles are not affected by the treatment. Fat from the destroyed fat cells is deposited into the extracellular fluid in the form of triglycerides. Under the influence of enzymes, it is broken up into glycerol and free fatty acids. The water-soluble glycerol enters the bloodstream and is used for energy while the insoluble fatty acids travel to the liver and are digested just like any other free fatty acid (including acids from food). The cavitation effect of ultrasound is only strong enough for therapeutic use in the frequency range between 20 kHz and 500 kHz. Since the applicator must be pressed against the skin during therapy, this increases possibility of transferring ultrasound energy into deeper levels of body, which is undesired and potentially harmful. Determining suitable subcutaneous treatment depth with an applicator without applying pressure represents a significant technical problem. In vacuum technology, there exist applicators which create a skin fold inside the interior of the applicator. If the same applicator contained an ultrasound source for the destruction of subcutaneous fat cells, it would be possible to perform both types of therapy simultaneously. This would not only create a sum of the effects of both therapies resulting in a shorter time period, but would also have important synergy effects. The principal design problem is the optimal ratio between the size of the ultrasound source and the size of the bell-shaped housing that creates the conditions required for a vacuum.
During therapy, the combination of negative pressure and ultrasound gel presents a problem. The gel is often used for easier entry of ultrasound into tissue, but the gel could also be absorbed into the vacuum part of the applicator, which is inconvenient.

### Prior state of the art

Ultrasound cavitation therapy is usually combined with endodermic vacuum massages. Until now, these therapies have been conducted one after another. There are currently several devices on the market that offer either deep therapy/massages or cavitation using ultrasound.

EP1260208 describes a device for circular deep vacuum therapy where a skin fold is formed in the applicator.

US 2008/0195000 A1 describes a system for non-invasive dermatological treatment using ultrasound energy combined with suction.

Devices that use high-frequency ultrasound between 1 and 30 MHz for diagnostic purposes are well-known. Several devices are also known that use ultrasound waves with frequencies between 1 and 5 MHz for thermal treatment of subcutaneous tissue. Devices also exist that use low-frequency ultrasound waves to break kidney stones. There are also known examples of devices that use low-frequency ultrasound to induce cavitation and destroy subcutaneous fat cells.

Devices that utilize the cavitation effect are manufactured by companies such as Ultrashepe ZDA, Liposonix and Bothell, all from the USA. The device from patent application WO2008114186A2 is also known.

The company Enraf Nonius srl (Netherland) produces a device that combines ultrasound with vacuum fixation. The weakness of the device in question is that the vacuum-inducing dish is made of rubber. During use, the rubber dish deforms and adheres to the skin. The device is based on ultrasound between 1 and 3 MHz and is stationary. The vacuum is used to fix the ultrasound electrodes to the tissue.

WO2008114186A2 describes cavitation therapy, but does not combine it with vacuum therapy.

EP1386597A1 and EP1219278A2 describe the use of ultrasound therapy in combination with vacuum therapy. From the shape and size of the ultrasound source, it is evident that the source is exclusively high-frequency. Low-frequency ultrasound sources have different design requirements. The disadvantage of these devices is that high frequency ultrasound does not achieve the same cavitation effect as low frequency ultrasound. The described system does not include a round bell shaped housing that would allow formation of a skin fold inside the cup and would be the most important part of radial deep therapy.

WO 2006/091093 describes a combination of high frequency ultrasound that does not include the low and medium frequency cavitation effect, and a deformable vacuum cup for fixation. It utilizes only the gel pads and cannot allow use of cheaper and commonly used ultrasound gel that is not solid, since it does not provide a solution for removal of excess liquefied gel via a filtration system in the handle.

A device manufactured by Emme srl (Italy) uses high-frequency ultrasound between 1 and 3 MHz in combination with deformable fixation vacuum cup. Due to the high ultrasound frequency, the cavitation effect is not strong enough for therapeutic use.

### Description of the solution to the technical problem

The low- and mid-frequency ultrasound device for cavitation in combination with radial deep skin therapy according to the invention has an ultrasound head built into a solid bell-shaped housing with the bottom side curved outward and a suitably smooth surface for easier movement across the skin. A unique design feature of the device according to the invention is that the ultrasound head is built into the bell-shaped housing in such a way that the therapeutic part of the ultrasound head reaches up to approximately one third of the height of the bell-shaped housing that enables vacuum therapy. Additionally, excess gel is removed via a filtration system in the handle.

The device according to the invention will be described in further detail using figures that show:

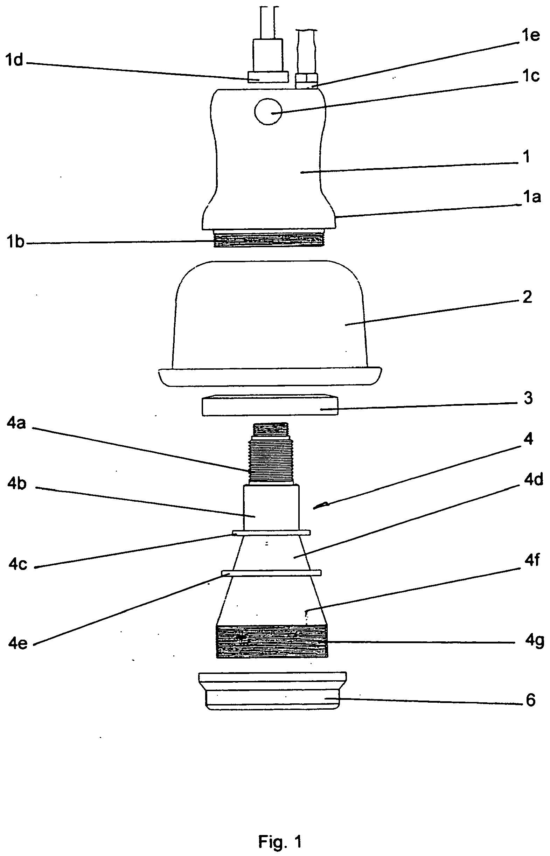

- Figure 1: - the device assembly

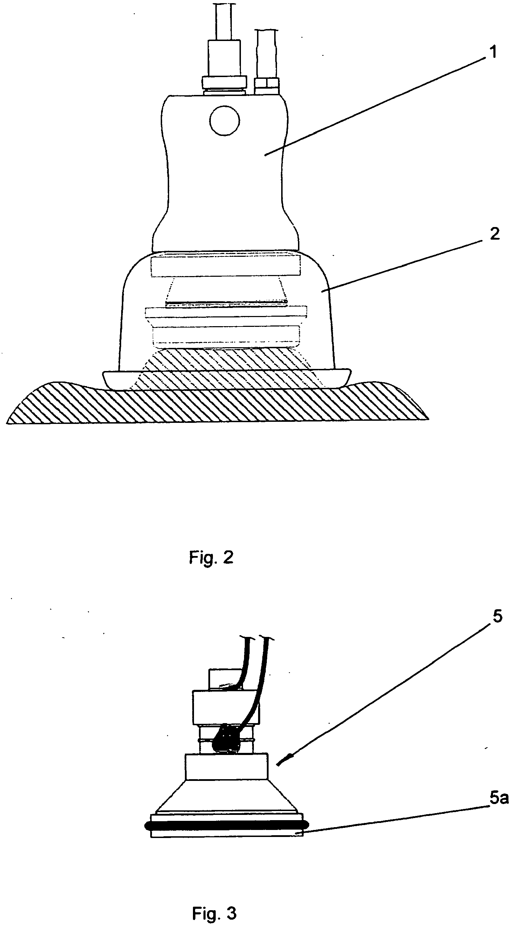

- Figure 2: - the device with a ring-shaped skin fold
- Figure 3: - ultrasound head 5

The device according to the invention has a roughly oval-shaped handle 1 that widens slightly in its lower part 1 a, followed by a narrower ring-shaped part 1 b with engraved threads. At the top of handle 1 is a round opening 1 d for the cables of the ultrasound head and opening 1e for the tube of the vacuum pump. In the upper part of handle 1, there is a round hole 1 c for a plug that can be used to admit air (decompress interior) for easier removal of the device at the end of the therapy.
A bell-shaped housing 2 is fastened to the narrow part 1 b using the inner thread of the fastening ring 3. Inside it is the cone-shaped housing of the ultrasound head 5 which is fixed to handle 1 using thread 4a. The space between parts 1 and 4 serves as a filter - reservoir for any absorbed impurities, especially ultrasound gel. The housing 4 is divided into three segments using rings 4c and 4e, which prevents gel from entering the interior of the device using the capillary effect.

Housing 4 consists of a cylindrical part 4a with engraved threads, a cylindrical part 4b with a ring-shaped part 4c, which continues into part 4d (shaped like a truncated cone). Between parts 4d and 4f, there is a ring-shaped part 4e. Part 4f is shaped like a truncated cone and ends at cylindrical part 4g with threads where the lid 6 is screwed after attaching the ultrasound head 5.

A unique design feature of the device according to the invention is that the ultrasound head 5 is built into the solid bell-shaped housing 2 in such a way that the therapeutic part of the head 5a reaches up to approximately one third of the height of the bell-shaped housing 2.

The device also comprises an attached or integrated electrical signal generator for ultrasound control and a vacuum pump for the purposes of vacuum therapy.

The device according to the invention combines ultrasound cavitation therapy with an endodermic vacuum massage, allowing the skin fold to be closer to ultrasound head 5 without putting pressure on any internal organs in the vicinity of the treated skin. Ultrasound head 5 can be made using the principle of a low-frequency ultrasound source in the 10-500 kHz range. There are several possible implementations of the ultrasound source, the most common being the piezoelectric crystal. It is possible to use a piezoelectric source where the piezoelectric crystal is surrounded by metal plates on the top and bottom. These plates act as a resonator.

Building the reservoir into the applicator itself solves the problem of ultrasound gel absorption. By using the capillary effect, the reservoir acts as a filtration system. Additionally, it is possible to use mechanical filters in the applicator, on the vacuum tube leading to the applicator, or inside the negative pressure pump. These filters prevent the ultrasound gel from being absorbed deeper into the vacuum system. Alternatively, conductive gel lining can be used. In this case, the gel is stored in a protective casing and thus not absorbed into the system.

The device according to variant I has tubes for the application of therapeutic ultrasound gel built into the handle 1. The gel is applied using a pump which can be controlled either manually or by a computer. An automatic sensor system can be integrated into the system for easier gel application. Medical or cosmetic substances can be added to the gel.

The device according to the invention can be made with a convex or concave shape of the lower part of the ultrasound source, the upper part of the ultrasound source or the piezoelectric crystal itself. Thus, the ultrasound wave can be focused or defocused.

The device according to variant II consists of several ultrasound sources spread over the surface of the ultrasound head. These ultrasound sources can be time- or phase-shifted. The sources can operate at the same frequency or at several different frequencies. One or more sources can operate in pulsed, continuous or modulated modes.

The device according to variant III includes one or more radiofrequency wave sources in handle 1. These allow skin heating or radiofrequency ablation.

The device according to variant IV includes an electronic circuit for estimation of skin depth and composition using a diagnostic ultrasound source. The system manually or automatically determines the optimal depth at which the system should operate and adjusts the parameters of the device accordingly.

The device according to variant V has the bell-shaped housing 2 shaped in such a way that the bottom side is oval.

The device according to variant VI has an extended holder on the handle, allowing better grip.

The device according to variant VII combines the ultrasound source in the bell-shaped housing 2 with one or more built-in balls, rollers, heating or cooling devices, electrical signal sources and/or sources of coherent or non-coherent light. The efficiency of a light source can be increased using materials that reflect light.

The device according to the invention can be used for intradermal or transdermal substance delivery, a phenomenon known as sonoporation. The advantages of the invention allow optimal effects in all points beneath the bell-shaped housing 2 and simultaneously ensure that moving the device over the patient's skin does not cause pain or discomfort.

## Claims

1. A low- and mid-frequency ultrasound device for cavitation in combination with radial deep skin therapy which comprises a handle (1), a fastening ring (3), a bell-shaped housing (2) fastened to the handle by said fastening ring (3), an ultrasound source (5), and a vacuum pump;
said device being adapted to perform low-frequency ultrasound cavitation therapy and vacuum therapy with a skin fold simultaneously;
**characterized in that,**
the ultrasound source is in a cone-shaped housing (4), said cone-shaped housing (4) is contained in said bell-shaped housing (2) and screwed to the handle, and
the space between said handle and said cone-shaped housing serves as a filter-reservoir for excess ultrasound gel absorbed into the device's interior during therapy .

2. The device according to claim 1 , **characterized in that** the handle (1) is approximately oval in shape and can slightly widen in its lower part (1a), which is followed by a narrower ring-shaped part (1b) with engraved threads, that the top of handle (1) has a round opening (1d) for the cables of the ultrasound head and an opening (1e) for the tube of the vacuum pump, that there is a round hole (1c) in the upper part of the handle for a plug that can be used to apply air for easier device removal (decompress interior) at the end of the therapy, that the bell-shaped housing (2) is fastened to the narrow part (1 b) using the inner thread of the fastening ring (3), that the cone-shaped housing (4) of the ultrasound head (5) screwed to the handle (1) using the thread (4a), that the housing (4) consists of a cylindrical part (4a) with engraved threads, a cylindrical part (4b) with a ring- shaped part (4c), which continues into truncated cone-shaped part (4d), that there is a ring-shaped part (4e) between parts (4d and 4f), that part (4f) is shaped like a truncated cone and ends with a cylindrical part (4g) with threads where the lid (6) is screwed after attaching the ultrasound head (5), that the ultrasound head (5) is built into the bell-shaped housing (2) in such a way that the therapeutic part (5a) reaches up to approximately one third of the height of the bell-shaped housing (2), that the housing (4) is divided into three segments using rings (4c and 4e), which keeps the gel from entering the interior of the device.

3. The device according to claims 1 and 2, **characterized in that** handle (1) has built-in tubes for ultrasound gel application during therapy.

4. The device according to claim 3, **characterized in that** the gel is applied using a pump.

5. The device according to claims 1 to 4, **characterized in that** several ultrasound sources are used, that these sources can be time- or phase-shifted, that the sources can operate at the same frequency or at different frequencies, that one or more sources can operate in pulsed, continuous or modulated modes.

6. The device according to claims 1 to 5, **characterized in that** handle (1) includes one or more radiofrequency wave sources that allow deep tissue heating or radiofrequency ablation.

7. The device according to claims 1 to 6, **characterized in that** it includes an electronic circuit for estimation of tissue depth and composition using a diagnostic ultrasound source.

8. The device according to claims 1 to 7, **characterized in that** the bell-shaped housing (2) is shaped in such a way that the bottom side is oval.

9. The device according to claims 1 to 8, **characterized in that** the ultrasound source is built into the bell-shaped housing (2) in combination with one or more balls, rollers, heating or cooling devices, electrical signal sources and/or sources of coherent or non-coherent light; that sources of light include materials that reflect light.

## Patentansprüche

1. Nieder- und mittelfrequente Ultraschallvorrichtung für Kavitation in Kombination mit radialer Hauttiefenbehandlung, die einen Handgriff (1), einen Befestigungsring (3), ein glockenförmiges Gehäuse (2), das an dem Handgriff mittels des Befestigungsrings (3) befestigt ist, eine Ultraschallquelle (5) und eine Vakuumpumpe umfasst;
wobei die Vorrichtung ausgeführt ist, niederfrequente Ultraschallkavitationstherapie und Vakuumtherapie an einer Hautfalte gleichzeitig auszuführen;
**dadurch gekennzeichnet, dass**,
die Ultraschallquelle sich in einem kegelförmigen Gehäuse (4) befindet, das kegelförmige Gehäuse (4) in dem glockenförmigen Gehäuse (2) enthalten und an dem Handgriff angeschraubt ist und
der Raum zwischen dem Handgriff und dem kegelförmigen Gehäuse als ein Filterbehälter für überschüssiges Ultraschallgel dient, das während der Therapie in das Vorrichtungsinnere absorbiert wird.

2. Vorrichtung nach Anspruch 1, **dadurch gekennzeichnet, dass** der Handgriff (1) in der Form annähernd oval ist und sich geringfügig in seinem unteren Teil (1a) weiten kann, dem ein engerer ringförmiger Teil (1b) mit eingeprägten Gewinden folgt, dass das Oberteil des Handgriffs (1) eine runde Öffnung (1d) für die Kabel des Ultraschallkopfes und eine Öffnung (1e) für das Rohr der Vakuumpumpe aufweist, dass es ein Rundloch (1c) in dem oberen Teil des Handgriffs für einen Stopfen gibt, der verwendet werden kann, um Luft für ein einfacheres Entfernen der Vorrichtung (dekomprimieren des Innenraums) am Ende der Therapie anzuwenden, dass das glockenförmige Gehäuse (2) an dem schmalen Teil (1b) unter Verwendung des Innengewindes des Befestigungsrings (3) befestigt ist, dass das kegelförmige Gehäuse (4) des Ultraschallkopfes (5) an dem Handgriff (1) unter Verwendung des Gewindes (4a) angeschraubt ist, dass das Gehäuse (4) aus einem Zylinderteil (4a) mit eingeprägten Gewinden, einem Zylinderteil (4b) mit einem ringförmigen Teil (4c), der sich in den abgeschnittenen kegelförmigen Teil (4d) fortsetzt, besteht, dass es einen ringförmigen Teil (4e) zwischen Teilen (4d und 4f) gibt, dass der Teil (4f) wie ein Kegelstumpf geformt ist und mit einem Zylinderteil (4g) mit Gewinden endet, an dem der Deckel (6) nach dem Befestigen des Ultraschallkopfes (5) angeschraubt wird, dass der Ultraschallkopf (5) in das glockenförmige Gehäuse (2) auf solche Art und Weise eingebaut ist, dass der therapeutische Teil (5a) bis zu annähernd einem Drittel der Höhe des glockenförmigen Gehäuses (2) reicht, dass das Gehäuse (4) unter Verwendung von Ringen (4c und 4e) in drei Segmente geteilt ist, was das Gel davon abhält, in das Innere der Vorrichtung einzudringen.

3. Vorrichtung nach den Ansprüchen 1 und 2, **dadurch gekennzeichnet, dass** der Handgriff (1) integrierte Rohre für die Ultraschallgelanwendung während der Therapie aufweist.

4. Vorrichtung nach Anspruch 3, **dadurch gekennzeichnet, dass** das Gel unter Verwendung einer Pumpe angewandt wird.

5. Vorrichtung nach den Ansprüchen 1 bis 4, **dadurch gekennzeichnet, dass** mehrere Ultraschallquellen verwendet werden, dass diese Quellen zeit - oder phasenverschoben sein können, dass die Quellen bei der gleichen Frequenz oder bei unterschiedlichen Frequenzen arbeiten können, dass eine oder mehrere Quellen in gepulsten, kontinuierlichen oder modulierten Modi arbeiten können.

6. Vorrichtung nach den Ansprüchen 1 bis 5, **dadurch gekennzeichnet, dass** der Handgriff (1) eine oder mehrere Hochfrequenzwellenquellen, die Tiefengewebeerwärmung oder Radiofrequenzablation ermöglichen, umfasst.

7. Vorrichtung nach den Ansprüchen 1 bis 6, **dadurch gekennzeichnet, dass** sie eine elektronische Schaltung zur Abschätzung von Gewebetiefe und -zusammensetzung unter Verwendung einer diagnostischen Ultraschallquelle umfasst.

8. Vorrichtung nach den Ansprüchen 1 bis 7, **dadurch gekennzeichnet, dass** das glockenförmige Gehäuse (2) auf solche Art und Weise geformt ist, dass die Unterseite oval ist.

9. Vorrichtung nach den Ansprüchen 1 bis 8, **dadurch gekennzeichnet, dass** die Ultraschallquelle in das glockenförmige Gehäuse (2) in Kombination mit einer oder mehreren Kugeln, Rollen, Erwärmungs- oder Kühlvorrichtungen, elektrischen Signalquellen und/oder Quellen von kohärentem oder nicht kohärentem Licht eingebaut ist;
dass Lichtquellen Materialien umfassen, die Licht reflektieren.

## Revendications

1. Dispositif à ultrasons basses et moyennes fréquences pour la cavitation en combinaison avec une thérapie de la peau profonde radiale, comprenant une poignée (1), un anneau de fixation (3), un boîtier en forme de cloche (2) fixé à la poignée par ledit anneau de fixation (3), une source d'ultrasons (5) et une pompe à vide ;
ledit dispositif étant adapté pour effectuer une thérapie de cavitation par ultrasons basses fréquences et une thérapie sous vide simultanément avec un pli cutané ;
**caractérisé en ce que**
la source d'ultrasons est un boîtier conique (4), ledit boîtier conique (4) étant contenu dans ledit boîtier en forme de cloche (2) et vissé à la poignée, et
l'espace entre ladite poignée et ledit boîtier conique sert de réservoir de filtre pour du gel ultrasonique excédentaire, absorbé à l'intérieur du dispositif pendant la thérapie.

2. Dispositif selon la revendication 1, **caractérisé en ce que** la poignée (1) présente une forme approximativement ovale et peut s'élargir légèrement dans sa partie inférieure (1a), laquelle est suivie d'une partie annulaire plus étroite (1b) avec des filets gravés, **en ce que** le haut de la poignée (1) présente une ouverture ronde (1d) pour les câbles de la tête à ultrasons, et une ouverture (1e) pour le tube de la pompe à vide, **en ce qu'**il est prévu un orifice rond (1c) dans la partie haute de la poignée, pour un bouchon susceptible d'être utilisé pour appliquer de l'air pour afin de faciliter le retrait du dispositif (intérieur décompressé) à la fin de la thérapie, **en ce que** le boîtier en forme de cloche (2) est fixé à la partie étroite (1b) à l'aide du filet intérieur de l'anneau de fixation (3), **en ce que** le boîtier conique (4) de la tête à ultrasons (5) est vissé à la poignée (1) à l'aide du filet (4a), **en ce que** le boîtier (4) consiste en une partie cylindrique (4a) avec des filets gravés, une partie cylindrique (4b) avec une partie annulaire (4c) suivie par une partie en forme de cône tronqué (4d), **en ce qu'**il est prévu une partie annulaire (4e) entre les parties (4d et 4f), **en ce que** la partie (4f) est formée comme un cône tronqué et se termine en une partie cylindrique (4g) avec des filets où le couvercle (6) est vissé après la fixation de la tête à ultrasons (5), **en ce que** la tête à ultrasons (5) est construite dans le boîtier en forme de cloche (2) de telle façon que la partie thérapeutique (5a) s'étend sur environ un tiers de la hauteur du boîtier en forme de cloche (2), **en ce que** le boîtier (4) est divisé en trois segments à l'aide des anneaux (4c et 4e), empêchant ainsi le gel de pénétrer à l'intérieur du dispositif.

3. Dispositif selon les revendications 1 et 2, **caractérisé en ce que** la poignée (1) comporte des tubes intégrés pour l'application de gel ultrasonique pendant la thérapie.

4. Dispositif selon la revendication 3, **caractérisé en ce que** le gel est appliqué à l'aide d'une pompe.

5. Dispositif selon les revendications 1 à 4, **caractérisé en ce que** plusieurs sources d'ultrasons sont utilisées, **en ce que** ces sources peuvent être décalées dans le temps ou en phase, **en ce que** les sources peuvent fonctionner à la même fréquence ou à des fréquences différentes, **en ce qu'**une ou plusieurs sources peuvent être utilisées dans des modes pulsé, continu ou modulé.

6. Dispositif selon les revendications 1 à 5, **caractérisé en ce que** la poignée (1) comprend une ou plusieurs sources d'ondes de hautes fréquences permettant de chauffer les tissus profonds ou d'effectuer une ablation par radiofréquence.

7. Dispositif selon les revendications 1 à 6, **caractérisé en ce qu'**il comprend un circuit électronique destiné à estimer la profondeur et la composition des tissus à l'aide d'une source d'ultrasons de diagnostic.

8. Dispositif selon les revendications 1 à 7, **caractérisé en ce que** le boîtier en forme de cloche (2) est formé de telle façon que le côté inférieur est ovale.

9. Dispositif selon les revendications 1 à 8, **caractérisé en ce que** la source d'ultrasons est construite dans le boîtier en forme de cloche (2) en combinaison avec un(e) ou plusieurs billes, rouleaux, dispositifs de chauffage ou de refroidissement, sources de signaux électriques et/ou sources de lumière cohérente ou non-cohérente ; **en ce que** les sources de lumière incluent des matériaux réfléchissant la lumière.
